(19) Europäisches Patentamt
European Patent Office
Office européen des brevets

(11) Publication number: 0 222 475 B1

(12) # EUROPEAN PATENT SPECIFICATION

(45) Date of publication of the patent specification: 29.11.89

(51) Int. Cl.[4]: C 07 C 143/833, C 07 D 209/08, C 07 D 317/62, C 07 D 319/18, C 07 D 307/79, A 61 K 31/64

(21) Application number: 86307158.5

(22) Date of filing: 17.09.86

(54) **Sulfonyl ureas with anti-tumour activity.**

(30) Priority: 23.09.85 US 779354
24.07.86 US 888675

(43) Date of publication of application: 20.05.87 Bulletin 87/21

(45) Publication of the grant of the patent: 29.11.89 Bulletin 89/48

(84) Designated Contracting States: AT BE CH DE FR GB IT LI LU NL SE

(56) References cited:
EP-A- 0 099 339
EP-A- 0 123 303
EP-A- 0 166 615
DE-B- 1 144 259
DE-B- 1 159 937
DE-B- 1 240 866
US-A- 3 097 242

(73) Proprietor: ELI LILLY AND COMPANY, Lilly Corporate Center, Indianapolis Indiana 46285 (US)

(72) Inventor: Howbert, James Jeffrey, 5720 North Ewing Street, Indianapolis Indiana 46220 (US)
Inventor: Poore, Gerald Auston, 1065 Old Moore Road, Martinsville Indiana 46151 (US)
Inventor: Rieder, Brent Jeffrey, 510 North State Street, Greenfield Indiana 46140 (US)
Inventor: Harper, Richard Waltz, 4470 North Delaware Street, Indianapolis Indiana 46250 (US)
Inventor: Tao, Eddie Vi-Ping, 1211 Kirkham Lane, Indianapolis Indiana 46260 (US)
Inventor: Aikins, James Abraham, 7731 Hallow Ridge Circle, Indianapolis Indiana 46256 (US)

(74) Representative: Tapping, Kenneth George et al, Lilly Industries Limited Patent Department Erl Wood Manor, Windlesham Surrey, GU20 6PH (GB)

EP 0 222 475 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Despite the development of numerous chemical agents and sophisticated regimens of drug therapy, cancer continues to extract an ever-increasing human toll of suffering and death. Although many advances have been made, especially in the area of combination drug therapy, the need for new and better methods of treating neoplasms and leukemias has not diminished. This is especially evident in the area of inoperable or metastatic solid tumors, such as various forms of lung cancer.

To be especially useful, new chemotherapeutic agents should have a wide spectrum of activity, a large therapeutic index, and be chemically stable and compatible with other agents. In addition, new agents having oral activity would be especially useful so that initial treatment and subsequent maintenance therapy can be made easily and without inconvenience or pain to the patient.

The present invention provides a series of sulfonylureas which are useful in the treatment of tumors. The compounds are orally active and relatively non-toxic providing an excellent therapeutic index.

Sulfonylureas are known in the art, particularly for their oral hypoglycemic utilities. See, for example, Chemical Abstracts 71:11457w (1969), Holland, et al., J. Med. Pharm. Chem., 3 (1), 99 (1961), Gandhi, et al., Arzneim.-Forsch., 21, 968 (1971), Rajagopalan, et al., J. Org. Chem., 30, 3369 (1965), and Petersen, Chem. Ber., 83, 551 (1950). In addition, some antimycotic activity is noted and the compounds have also been prepared as derivatives of carbodiimides. A general review of compounds of this structural type may be found in Kurzer, Chem. Rev., 50, 1 (1952). Bicyclic sulfonylureas also are known in the art as hypoglycemic agents; See, e.g., Chemical Abstracts 67:54036t (abstracting German OLS 1 249 866), U.S. Patent 3 097 242, Chemical Abstracts 60:9220h (abstracting German OLS 1 159 937), and Lerner, et al., Metab., Clin. Explt., 14 (5), 578 (1965). These references, however, do not disclose any anti-tumor activity of these compounds.

Thus, in accordance with the invention, sulfonylurea derivatives of formula (I):

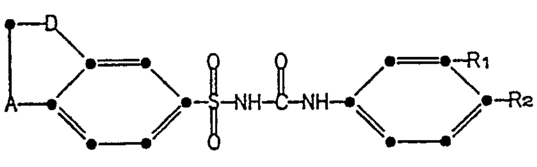

(I)

in which

|

A is -O-, $-NCH_3$, $-CH_2-$, $-CH_2CH_2-$, or $-CH_2O-$;

D is $-CH_2-$ or -O-;

$R_1$ is hydrogen or halo; and

$R_2$ is halo or trifluoromethyl, are useful as antineoplastic agents.

A method for treating susceptible neoplasms in a warm-blooded animal comprises administering to said animal a compound of formula (I).

In addition, this invention provides pharmaceutical formulations comprising as an active ingredient a compound of formula (I) in combination with a pharmaceutical-acceptable carrier, diluent, or excipient therefor. These formulations are particularly useful in treating mammals suffering from susceptible neoplasms.

The term «halo» refers to fluoro, chloro, bromo, and iodo. The term «$C_1$-$C_3$ alkyl» refers to methyl, ethyl, propyl, and isopropyl.

The preferred compounds of this invention are those of formula (I) in which

a) $R_1$ is hydrogen,

b) $R_2$ is halo, especially bromo, chloro, or fluoro, or trifluoromethyl, and

c) A is $-CH_2-$.

The most preferred compound of this invention is N-([(4-chlorophenyl)amino]carbonyl)-2,3-dihydro-1H-indene-5-sulfonamide.

The compounds of formula (I) generally are referred to as derivatives of N-([(-substituted phenyl)amino]carbonyl)arylsulfonamides, for example, as used in the previous paragraph. Alternatively, the compounds are referred to as 1-(substituted phenyl)-3-(arylsulfonyl)ureas.

The compounds of formula (I) may be prepared by any number of methods known in the literature. These methods are summarized by Kurzer, Chem. Rev., 50, 1 (1952), especially pages 4-19. Specific reference describing processes that can be employed in the preparation of compounds of formula (I) are those previously described in the reference cited above. All of these reference are expressly incorporated into this application by reference.

A preferred method of preparing the compounds of formula (I) is that of the reaction of a sulfonylisocyanate of formula (II):

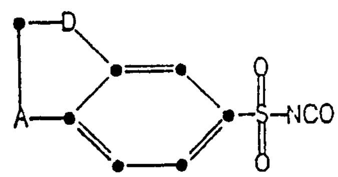

(II)

with an aniline derivative of the formula (III):

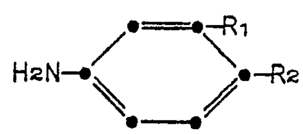

(III)

where A, D, $R_1$, and $R_2$ are as previously defined.

The reaction between compounds II and III is usually performed using equimolar amounts of the two reactants, although other ratios are operative. The reaction usually is carried out in an aprotic nonreactive solvent such as benzene, toluene, acetonitrile, diethyl ether, tetrahydrofuran, dioxane, or preferably methylene chloride. The reaction may be carried out at temperatures from about 0°C up to the boiling point of the reaction mixture. At the preferred temperature range of about 20-30°C, the reaction produces a strong exotherm and the reaction usually is complete within 1 hour. The product obtained may be recovered by filtration and may be purified, if desired, by any number of methods known to those skilled in the art, such as chromatography or crystallization.

Alternatively, an appropriately substituted sulfonamide of formula (IV):

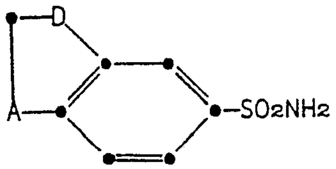

(IV)

may be reacted with an isocyanate of formula (V):

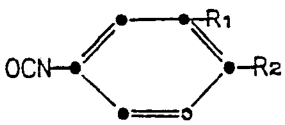

(V)

to provide the compounds of formula (I). The reaction generally is carried out in a water miscible, non-reactive solvent such as tetrahydrofuran or acetone. Generally, an equimolar amount or slight molar excess of the formula (V) compound is employed, although other ratios are operative. In addition, an aqueous solution of a base, such as sodium or potassium hydroxide, is employed. Usually the amount of base used is imately equimolar to the amount of compound (IV). The reaction generally is carried out from about 0°C up to the boiling point of the reaction mixture. At the preferred temperature range of 20 to 30°C, the reaction usually is complete within about three days.

Another preferred method of preparing compounds of formula (I) involves the reaction of sulfonamide (IV) with an alkyl haloformate to provide carbamate (VI) which is then reacted with aniline (III) to provide the corresponding product (I)

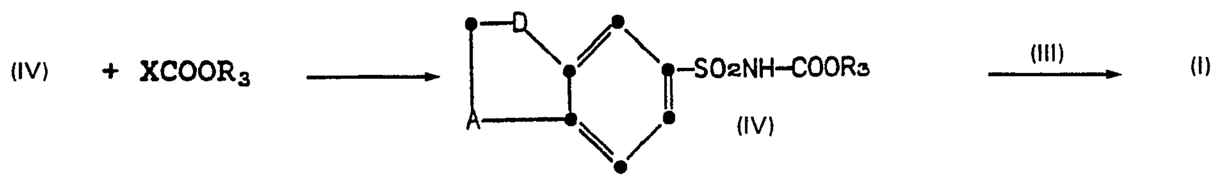


where X is bromo or chloro and $R_3$ is $C_1$-$C_3$ alkyl. The transformation of compound (IV) into compound (VI) usually is accomplished in a non-reactive solvent, such as acetone or methyl ethyl ketone, in the presence of an acid scavenger, such as an alkali metal carbonate, for example potassium carbonate. A molar excess of the haloformate usually is added, although other ratios are operative, and the reaction mixture heated at a temperature from about 30°C up to the reflux temperature for a period of 1-6 hours to provide the desired intermediate (VI). Intermediate carbamate (VI) and aniline (III) then are heated together in an inert high-boiling solvent, such as dioxane, toluene, or diglyme, at temperatures from about 50°C up to the reflux temperature of the mixture to provide the desired product.

Thus, in a further aspect of the invention, there is provided a process for preparing a compound of formula (I), as defined above, which comprises reacting with a compound of the formula

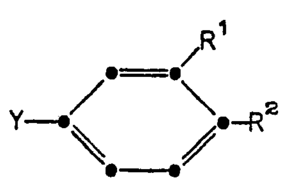

in which Y is $-NH_2$ or -NCO, and $R^1$ and $R^2$ are as defined above, a sulfonyl compound of the formula:

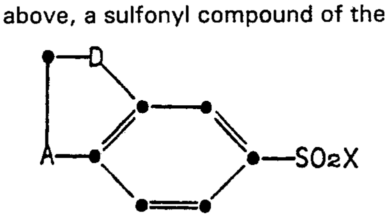

in which X is -NCO, $-NH_2$ or $-NH-COOR_3$ in which $R_3$ is $C_1$-$C_3$ alkyl, and A and D are as defined above, provided that if X is -NCO or $-NHCOOR_3$ then Y is $-NH_2$ and if X is $-NH_2$ then Y is -NCO.

Intermediates (II), (III), (IV), and (V) and any other reagents required for other methods of preparation, are either commercially available, are known in the literature, or can be prepared by methods known in the art.

Certain intermediates of formula (IV), especially those in which A and D are, independently, -O- or $-CH_2-$, may be prepared by chlorosulfonating an appropriately substituted benzene compound at 50° to 130°C with a Villsmeier reagent prepared from sulfuryl chloride and dimethylformamide followed by ammonolysis with ammonia or ammonium hydroxide. Examples 7A and 8 below illustrate this process.

The following non-limiting examples are provided to further illustrate the invention.

*Example 1*

N-([(4-chlorophenyl)amino]carbonyl)-2,3-dihydro-1H-indene-5-sulfonamide

To a solution of 93.2 g of 2,3-dihydro-5-indenylsulfonamide in 300 ml of acetone were added 490 ml of a 1N sodium hydroxide solution. A solution of 79.36 g of 4-chlorophenylisocyanate in 250 ml of acetone was added to the reaction mixture with stirring. After stirring at room temperature for 18 hours, the reaction mixture was filtered and 490 ml of 1N hydrochloric acid were added to the filtrate thereby providing a fine white precipitate. One liter of water was added, and the solid was recovered by filtration to provide 144.86 g of the desired title product, m.p. 169 to 172°C.

Analysis for $C_{16}H_{15}ClN_2O_3S$:

| | | | |
|---|---|---|---|
| Calculated: | C, 54.78; | H, 4.31; | N, 7.79; |
| Found: | C, 54.95; | H, 4.43; | N, 7.94. |

*Example 2*

N-([(4-chlorophenyl)amino]carbonyl)-5,6,7,8-tetrahydro-2-naphthalenesulfonamide

The title compound was prepared by the method of Example 1 in 56% yield from 5,6,7,8-tetrahydro-2-naphthalenesulfonamide and 4-chlorophenylisocyanate, m.p. 163-165°C.

Analysis for $C_{17}H_{17}ClN_2O_3S$:

| | | | | |
|---|---|---|---|---|
| Calculated: | C, 55.96; | H, 4.70; | N, 7.68; | S, 8.79; |
| Found: | C, 55.91; | H, 4.62; | N, 7.56; | S, 9.00. |

*Example 3*

Alternate preparation of N-([(4-chlorophenyl)amino]carbonyl)-2,3-dihydro-1H-indene-5-sulfonamide

A. Preparation of [(2,3-dihydro-1H-inden-5-yl)-sulfonyl]carbamic acid ethyl ester.

Two hundred eighty grams of potassium carbonate were added to a solution of 181.4 g of 2,3-dihydro-5-indenylsulfonamide in three liters of methyl ethyl ketone. The suspension was stirred for 45 minutes at which time 98 ml of ethyl chloroformate were added in dropwise manner. After stirring for one hour at room temperature, the mixture was heated to reflux and stirred an additional three hours. After cooling, the mixture was added to ice water, filtered, brought to a pH of 1, and extracted three times with ethyl acetate. The combined organic extracts were washed with water and a saturated sodium chloride solution, dried over sodium sulfate, and evaporated to dryness in vacuo. Crystallization of the residue from toluene provided 176.2 g of the desired subtitle intermediate, m.p. 92-95°C.

Analysis for $C_{12}H_{15}NO_4S$:

| | | | |
|---|---|---|---|
| Calculated: | C, 53.52; | H, 5.61; | N, 5.20; |
| Found: | C, 53.76; | H, 5.71; | N, 5.08. |

B. Preparation of N-([(4-chlorophenyl)amino]carbonyl)-2,3-dihydro-1H-indene-5-sulfonamide.

A solution of 2.69 g of [(2,3-dihydro-1H-inden-5-yl)sulfonyl]carbamic acid ethyl ester and 1.27 g of 4-chloroaniline in 50 ml of dioxane was heated at reflux for 16 hours under a nitrogen atmosphere. The solution was added to water and extracted with ethyl acetate. The organic extract was dried over sodium sulfate and concentrated in vacuo giving a crystalline solid. The solid was triturated with toluene and filtered to provide 1.6 g of the desired title product, m.p. 175-177°C.

Analysis for $C_{16}H_{15}ClN_2O_3S$:

| | | | |
|---|---|---|---|
| Calculated: | C, 54.78; | H, 4.31; | N, 7.79; |
| Found: | C, 54.63; | H, 4.47; | N, 7.84. |

*Example 4*

N-([(4-chlorophenyl)amino]carbonyl)-2,3-dihydro-5-benzofuransulfonamide

The title product was prepared in 26.1% yield from 2,3-dihydro-5-benzofuransulfonamide and 4-chlorophenylisocyanate following the procedure of Example 1, m.p. 190-194°C.

Analysis for $C_{15}H_{13}ClN_2O_4S$:

| | | | | |
|---|---|---|---|---|
| Calculated: | C, 51.07; | H, 3.71; | N, 7.94; | S, 9.07; |
| Found: | C, 51.32; | H, 4.00; | N, 7.73; | S, 9.02. |

*Example 5*

N-([(4-chlorophenyl)amino]carbonyl)-2,3-dihydro-1-methyl-1H-indole-5-sulfonamide

Following the procedure of Example 3B, the title product was prepared in 60% yield from [(2,3-dihydro-1-methyl-1H-indol-5-yl)sulfonyl]carbamic acid ethyl ester and 4-chloroaniline, m.p. 145-147°C.

Analysis for $C_{16}H_{16}ClN_3O_3S$:

| | | | | |
|---|---|---|---|---|
| Calculated: | C, 52.53; | H, 4.41; | N, 11.49; | S, 8.76; |
| Found: | C, 52.78; | H, 4.47; | N, 11.19; | S, 8.56. |

*Example 6*

N-([(3,4-dichlorophenyl)amino]carbonyl)-2,3-dihydro-1H-indene-5-sulfonamide

A solution of 2.67 g of 3,4-dichloraniline in 10 ml of toluene was added to 3.87 g of 2,3-dihydro-5-indenylsulfonylisocyanate in 20 ml of toluene. After stirring 7 hours, the resulting precipitate was recovered by filtration, washed with toluene and dried providing 5.38 g of the title product, m.p. 155.5 to 158°C.

Analysis for $C_{16}H_{14}Cl_2N_2O_3S$:

| | | | | |
|---|---|---|---|---|
| Calculated: | C, 49.88; | H, 3.66; | N, 7.27; | S, 8.32; |
| Found: | C, 50.13; | H, 3.84; | N, 7.31; | S, 8.05. |

*Example 7*

N-([(4-chlorophenyl)amino]carbonyl)-1,3-benzodioxole-5-sulfonamide

A. Preparation of 1,3-benzodioxole-5-sulfonamide.

A 500 ml 3-neck round bottom flask was charged with 38.7 g (0.52 mole) of dimethylformamide. The contents of the flask were cooled to 0°C. After cooling, 70.18 g (0.52 mole) of sulfuryl chloride were added and the contents of the flask stirred for 10 minutes while maintaining the temperature at approximately 10°C.

After the Villsmeier reagent was formed, 61.06 g (0.5 mole) of 1,3-benzodioxole were added over a period of 5 minutes. The mixture was heated to 80°C for approximately 10 minutes. The temperature was increased to 110°C, and maintained for 5 minutes. The reaction mixture was allowed to cool to 40°C and poured into a mixture of 450 g crushed ice, 200 ml water, and 200 ml of chloroform.

The resulting organic layer was decanted and then dripped into 200 ml of concentrated ammonium hydroxide. The solution was stirred for approximately $1^1/_2$ hours. After stirring, the organic and aqueous phases were allowed to separate and a yellow granular precipitate formed at the interface of the two layers. This solid was collected by filtration, washed with 100 ml of water, and dried overnight at 40°C to provide 26.9 g of the desired subtitle intermediate, m.p. 158-160°C. Both mass spectroscopy and NMR spectra were consistent with the structure of the desired intermediate.

B. Preparation of N-([(4-chlorophenyl)amino]-carbonyl)-1,3-benzodioxole-5-sulfonamide.

The title product was prepared in 75% yield from the intermediate of Example 7A and 4-chlorophenylisocyanate following the procedure of Example 1.

Analysis for $C_{14}H_{11}ClN_2O_5S$:
Calculated: C, 47.40; H, 3.13; N, 7.90;
Found: C, 47.54; H, 3.23; N, 8.10.

*Example 8*

N-([(4-chlorophenyl)amino]carbonyl)-2,3-dihydro-1,4-benzodioxin-6-sulfonamide

Following the procedure of Example 7A, 1,4-benzodioxan was transformed into 1,4-benzodioxan-6-sulfonamide in 34% yield. This sulfonamide was then converted into the title sulfonylurea in 66% yield according to the procedure of Example 1, m.p. 191°C.

Analysis for $C_{15}H_{13}ClN_2O_5S$:
Calculated: C, 48.85; H, 3.55; N, 7.60;
Found: C, 48.57; H, 3.75; N, 7.40.

The compounds of formula I have been shown to be active against transplanted mouse tumors in vivo. The compounds are active in the test systems when administered according to a variety of dosage schedules. In general, the compounds were administered orally daily or twice daily for 8-10 days.

To demonstrate the anti-tumor activity of the compounds of formula I, the compounds were tested in animals bearing a 6C3HED lymphosarcoma, also known as the Gardner lymphosarcoma (GLS). Table 1 gives the results of several experiments in mice bearing this tumor when compounds were administered orally. In the Table, column 1 gives the example number of the compound; column 2, the dose level; and column 3, the percent inhibition of tumor growth. The results are the average of 10 animals per group as compared with a suitable control group.

TABLE 1

Activity of the Compounds of Formula I against the 6C3HED Lymphosarcoma*

| Compound of Example No. | Dose** | Percent Inhibition |
|---|---|---|
| 1 | 150 | 91 |
| | 300 | 99 |
| | 50 twice daily | 65 |
| | 75 twice daily | 89 |
| | 100 twice daily | 93 |
| | 150 twice daily | 100 |
| | 200 twice daily | 99 |
| | 300 twice daily | 100 |
| | 400 twice daily | 100 |
| 2 | 150 | 38 |
| | 300 | 78 |
| 4 | 150 | 71 |
| | 300 | 94 |
| 5 | 150 | 71 |
| | 300 | 100 |
| 6 | 150 | 74 |
| | 300 | 97 |
| 7 | 150 | 85 |
| | 300 | 100 |
| 8 | 150 | 38 |
| | 300 | 49 |

* Tested in C3H mice.

** mg/kg administered orally in emulphor. Dosing began the day following inoculation. Compounds were dosed once every day for eight days, except where noted.

In addition, one of the compounds of formula (I), the compound of Example 1, was tested orally in additional test systems. These include the subcutaneous B-16 melanoma (B16-sc), the X5563 plasma cell myeloma (X5563), the M-5 ovarian carcinoma (M-5), the C3H mammary carcinoma (C3H), colon carcinoma-26 (C6), the CA-755 adenocarcinoma (CA755), the Madison Lung Carcinoma (Madison), the P388 lymphocytic leukemia (P388), and the Lewis Lung carcinoma (LL). A summary of these test results is provided in Table 2.

TABLE 2

Activity of Compound 1 against a variety of tumor models

| Compound of Example No. | Tumor | Dose* | Percent Inhibition |
|---|---|---|---|
| 1 | CA755 | 37.5 | 30 |
| | | 75.0 | 67 |
| | | 150 | 91 |
| | | 300 | 99 |
| | LL | 37.5 | 4 |
| | | 75 | 36 |
| | | 150 | 37 |
| | | 300 | 58 |
| | C6 | 37.5 | 24 |
| | | 75.0 | 36 |
| | | 150 | 69 |
| | | 300 | 85 |
| | | 600 | 100 |

TABLE 2 (continued)

| Compound of Example No. | Tumor | Dose* | Percent Inhibition |
|---|---|---|---|
| | M-5 | 37.5 | 53 |
| | | 75.0 | 76 |
| | | 150 | 88 |
| | | 300 | 96 |
| | | 600 | 99 |
| | Madison | 37.5 | 44 |
| | | 75.0 | 55 |
| | | 150 | 61 |
| | | 300 | 80 |
| | X5563** | 37.5 | 0-38 |
| | | 75 | 38-41 |
| | | 150 | 50-54 |
| | | 300 | 46-66 |
| | C3H | 37.5 | 49 |
| | | 75 | 90 |
| | | 150 | 96 |
| | | 300 | 100 |
| | P388 | 12.5 | 10*** |
| | | 25 | 8*** |
| | | 50 | 13*** |
| | | 100 | 19*** |
| | | 200 | 51*** |
| | B16-sc | 37.5 | 13 |
| | | 75 | 13 |
| | | 150 | 3 |
| | | 300 | 25 |

* mg/kg per dose administered orally in emulphor. Dosing began the day following inoculation. Compounds were dosed twice daily for 10 days.

** Summary of two experiments.

*** Percent prolongation of life.

The compounds of formula (I) are antineoplastic agents; a method of treating susceptible neoplasms comprising administering a compound of formula (I) by various routes including the oral, rectal, transdermal, subcutaneous, intravenous, intramuscular, or intranasal routes, being usually employed in the form of a pharmaceutical composition. A special feature of these compounds is that they are effective when administered orally. Such compositions are prepared in a manner well-known in the pharmaceutical art and comprise at least one active compound of formula (I). Accordingly, in addition to providing compounds of formula I, the invention also provides pharmaceutical compositions comprising as active ingredient a compound of formula I associated with a pharmaceutically acceptable carrier.

In making the compositions of the present invention, the active ingredient usually will be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates,talc, magnesium stearate and mineral oil. The formulations can include additionally lubricating agents, wetting agents, emulsifying and suspending agents, preserving agents, sweetening agents or flavoring agents. The compositions of the invention may be formulated so as to provide quick, sustained or delayed release of the active ingredient, after administration to the patient, by employing procedures well known in the art.

The compositions preferably are formulated in a unit dosage form, each dosage containing from about 5 to about 500 mg, more usually about 25 to about 300 mg, of the active ingredient. The term «unit dosage form» refers to physically discrete units suitable as unitary dosages for human subjects or other warm-blooded animals, each unit containing a predetermined quantity of active material calculated to produce the desired therapeutic effect, in association with a suitable pharmaceutical carrier.

The active compounds are effective over a wide dosage range. For example, dosages per day will normally fall within the range of about 0.5 to about 600 mg/kg of body weight. In the treatment of adult humans, the range of about 1 to about 50 mg/kg, in single or divided doses, is preferred. However, the amount of the compound actually administered will be determined by a physician, in the light of the relevant circumstances including the condition to be treated, the choice of compound to be administered, the chosen route of administration, the age, weight, and response of the individual patient, and the severity of the patient's symptoms. Therefore, the above dosage ranges are not intended to limit the scope of the invention in any way.

The following non-limiting formulation examples may employ as active compounds any of the compounds of formula (I) and are provided for illustrative purposes only.

*Example 9*

Hard gelatin capsules are prepared using the following ingredients:

| | *Quantity (mg/capsule)* |
|---|---|
| N-([(4-trifluoromethylphenyl)-amino]carbonyl)-2,3-dihydro-1,4--benzodioxin-6-sulfonamide | 250 |
| Starch dried | 200 |
| Magnesium stearate | 10 |

The above ingredients are mixed and filled into hard gelatin capsules in 460 mg quantities.

*Example 10*

A tablet formula is prepared using the ingredients below:

| | Quantity (mg/tablet) |
|---|---|
| N-([(3-fluoro-4-trifluoro-methylphenyl)amino]carbonyl)-2,3--dihydro-1H-indene-5-sulfonamide | 250 |
| Cellulose, microcrystalline | 400 |
| Silicon dioxide, fumed | 10 |
| Stearic acid | 5 |

The components are blended and compressed to form tablets each weighing 665 mg.

*Example 11*

An aerosol solution is prepared containing the following components:

| | Weight % |
|---|---|
| N-([(3-chloro-4-fluorophenyl)-amino]carbonyl)-5,6,7,8-tetra-hydro-2-naphthalenesulfonamide | 0.25 |
| Ethanol | 29.75 |
| Propellant 22 (Chlorodifluoromethane) | 70.00 |

The active compound is mixed with ethanol and the mixture added to a portion of the propellant 22, cooled to −30°C and transferred to a filling device. The required amount is then fed to a stainless steel container and diluted with the remainder of the propellant. The valve units are then fitted to the container.

*Example 12*

Tablets each containing 60 mg of active ingredient are made up as follows:

| | |
|---|---|
| N-([(4-chlorophenyl)amino]-carbonyl)-2,3-dihydro-1H--indene-5-sulfonamide | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which then are passed through a No. 14 mesh U.S. sieve. The granules so produced are dried at 50-60°C and passed through a No. 18 mesh U.S. sieve. The sodium carboxymethyl starch, magnesium stearate and talc, previously passed through a No. 60 mesh U.S. sieve, then are added to the granules which, after mixing, are compressed on a tablet machine to yield tablets each weighing 150 mg.

*Example 13*

Capsules each containing 80 mg of medicament are made as follows:

| | |
|---|---|
| N-([(4-fluorophenyl)amino]-carbonyl)-2,3-dihydro-1H--indene-5-sulfonamide | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

*Example 14*

Suppositories each containing 225 mg of active ingredient are made as follows:

| | |
|---|---|
| N-([(3,4-dichlorophenyl)amino]-carbonyl)-5,6,7,8-tetrahydro--2-naphthalenesulfonamide | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture then is poured into a suppository mold of nominal 2 g capacity and allowed to cool.

*Example 15*

Suspensions each containing 50 mg of medicament per 5 ml dose are made as follows:

| | |
|---|---|
| N-([(3,4-difluorophenyl)amino]-carbonyl)-2,3-dihydro-1H-indene--5-sulfonamide | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to | 5 ml |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

*Example 16*

Capsules each containing 150 mg of medicament are made as follows:

| | |
|---|---|
| N-([(4-chlorophenyl)amino]-carbonyl)-2,3-dihydro-1H--indene-5-sulfonamide | 150 mg |
| Starch | 164 mg |
| Microcrystalline cellulose | 164 mg |
| Magnesium stearate | 22 mg |
| Total | 500 mg |

The active ingredient, cellulose, starch and magnesium stearate are blended, passed through a No. 45 mesh U.S. sieve, and filled into hard gelatin capsules in 500 mg quantities.

**Claims for the Contracting States:**
BE, CH, DE, FR, GB, IT, LI, LU, NL, SE

1. A compound of formula (I):

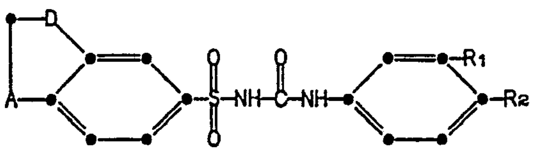 (I)

in which

A is -O-, $-NCH_3$, $-CH_2-$, $-CH_2CH_2-$, or $-CH_2O-$;
D is $-CH_2-$ or -O-;
$R_1$ is hydrogen or halo, and
$R_2$ is halo or trifluoromethyl; provided that when A is oxygen, D is $-CH_2-$ and $R_1$ is hydrogen, $R_2$ is not chloro.

2. A compound of formula (I), as claimed in claim 1 which is

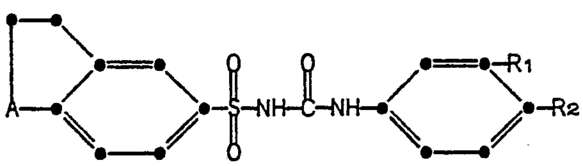

in which
A is $-CH_2-$ or $-CH_2CH_2-$,
$R_1$ is hydrogen or halo, and
$R_2$ is halo or trifluoromethyl.

3. A compound as claimed in claim 1 or 2 in which $R_1$ is hydrogen.

4. A compound as claimed in claim 3 in which $R_2$ is halo.

5. A compound as claimed in claim 4 in which $R_2$ is chloro.

6. N-([(4-chlorophenyl)amino]carbonyl)-2,3-dihydro-1H-indene-5-sulfonamide,
N-([(4-chlorophenyl)amino]carbonyl)-5,6,7,8-tetrahydro-2-naphthalenesulfonamide, or
N-([(4-chlorophenyl)amino]carbonyl)-1,3-benzodioxole-5-sulfonamide.

7. A compound of formula (I), as defined in claim 1, for use as a therapeutic agent.

8. A compound of formula (I), as defined in claim 1, for use as an antineoplastic agent.

9. A pharmaceutical formulation which comprises as an active ingredient, a compound of formula (I), as defined in claim 1, associated with a pharmaceutically-acceptable carrier, diluent or excipient therefor.

10. A process for preparing a compound of formula (I), as defined in claim 1, which comprises reacting with a compound of the formula

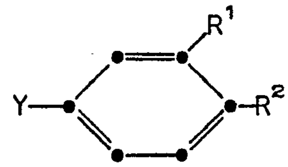

in which Y is $-NH_2$ or -NCO, and $R^1$ and $R^2$ are as defined above, a sulfonyl compound of the formula:

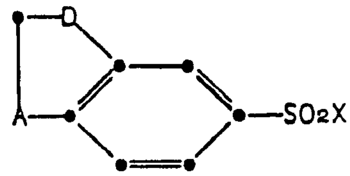

in which X is -NCO, $-NH_2$ or $-NH-COOR_3$ in which $R_3$ is $C_1$-$C_3$ alkyl, and A and D are as defined above, provided that if X is -NCO or $-NHCOOR_3$ then Y is $-NH_2$ and if X is $-NH_2$ then Y is -NCO.

11. Use of a compound of formula (I)

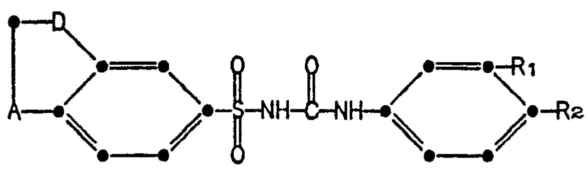

in which

A is -O-, $-NCH_3$, $-CH_2-$, $-CH_2CH_2-$, or $-CH_2O-$;
D is $-CH_2-$ or -O-;
$R_1$ is hydrogen or halo, and
$R_2$ is halo or trifluoromethyl, for the manufacture of an anti-tumor medicament.

**Claims for the Contracting State:** AT

1. A process for preparing a compound of formula (I):

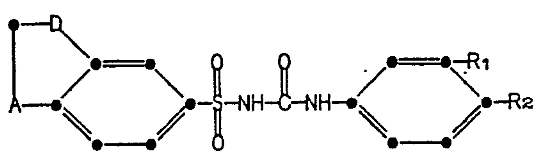 (I)

in which

A is -O-, $-NCH_3$, $-CH_2-$, $-CH_2CH_2-$, or $-CH_2O-$;
D is $-CH_2-$ or -O-;
$R_1$ is hydrogen or halo, and
$R_2$ is halo or trifluoromethyl, provided that when A is oxygen, D is $-CH_2-$ and $R_1$ is hydrogen, $R_2$ is not chloro, which comprises reacting with a compound of the formula

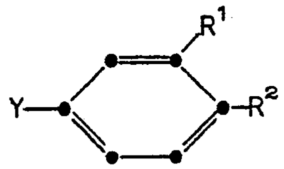

in which Y is $-NH_2$ or -NCO, and $R^1$ and $R^2$ are as defined above, a sulfonyl compound of the formula:

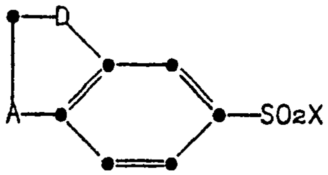

in which X is -NCO, $-NH_2$ or $-NH-COOR_3$ in which $R_3$ is $C_1$-$C_3$ alkyl, and A and D are as defined above, provided that if X is -NCO or $-NHCOOR_3$ then Y is $-NH_2$ and if X is $-NH_2$ then Y is -NCO.

2. A process for preparing a compound of formula (I), as claimed in claim 1 which is

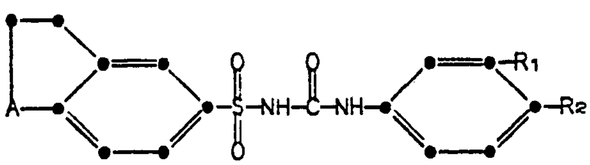

in which
A is $-CH_2-$ or $-CH_2CH_2-$,
$R_1$ is hydrogen or halo, and
$R_2$ is halo or trifluoromethyl.

3. A process for preparing a compound as claimed in claim 1 or 2 in which $R_1$ is hydrogen.

4. A process for preparing a compound as claimed in claim 3 in which $R_2$ is halo.

5. A process for preparing a compound as claimed in claim 4 in which $R_2$ is chloro.

6. A process as claimed in claim 1 for preparing N-([(4-chlorophenyl)amino]carbonyl)-2,3-dihydro-1H-indene-5-sulfonamide,
N-([(4-chlorophenyl)amino]carbonyl)-5,6,7,8-tetrahydro-2-naphthalenesulfonamide, or
N-([(4-chlorophenyl)amino]carbonyl)-1,3-benzodioxole-5-sulfonamide.

**Patentansprüche für die Vertragsstaaten:**
BE, CH, DE, FR, GB, IT, LI, LU, NL, SE

1. Verbindung der Formel (I)

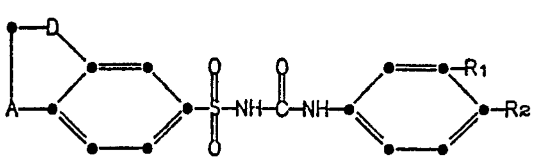
(I)

worin
A für -O-, $-NCH_3$, $-CH_2-$, $-CH_2CH_2-$ oder $-CH_2O-$ steht,
D für $-CH_2-$ oder -O- steht,
$R_1$ Wasserstoff oder Halogen ist, und
$R_2$ Halogen oder Trifluormethyl bedeutet,
mit der Maßgabe, daß $R_2$ nicht Chlor bedeutet, falls A Sauerstoff ist, D für $-CH_2-$ steht und $R_1$ Wasserstoff darstellt.

2. Verbindung der Formel (I) gemäß Anspruch 1, nämlich der Formel

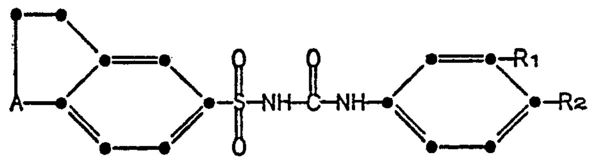

worin
A für $-CH_2-$ oder $-CH_2CH_2-$ steht,
$R_1$ Wasserstoff oder Halogen ist, und
$R_2$ Halogen oder Trifluormethyl bedeutet.

3. Verbindung nach Anspruch 1 oder 2, dadurch gekennzeichnet, daß $R_1$ Wasserstoff ist.

4. Verbindung nach Anspruch 3, dadurch gekennzeichnet, daß $R_2$ Halogen ist.

5. Verbindung nach Anspruch 4, dadurch gekennzeichnet, daß $R_2$ Chlor ist.

6. N-([(4-Chlorophenyl)amino]carbonyl)-2,3-dihydro-1H-inden-5-sulfonamid,
N-([(4-Chlorphenyl)amino]carbonyl-5,6,7,8-tetrahydro-2-naphthalinsulfonamid oder
N-([(4-Chlorphenyl)amino]carbonyl)-1,3,-benzodioxol-5-sulfonamid.

7. Verbindung der Formel (I) gemäß Definition von Anspruch 1 zur Verwendung als therapeutisches Mittel.

8. Verbindung der Formel (I) gemäß Definition nach Anspruch zur Verwendung als antineoplastisches Mittel.

9. Pharmazeutische Formulierung, dadurch gekennzeichnet, daß sie eine Verbindung der Formel (I) gemäß Definition nach Anspruch 1 als Wirkstoff in Kombination mit einem pharmazeutisch annehmbaren Träger, Verdünnungsmittel oder Hilfsstoff hierfür enthält.

10. Verfahren zur Herstellung einer Verbindung der Formel (I) gemäß Definition nach Anspruch 1, dadurch gekennzeichnet, daß eine Verbindung der Formel

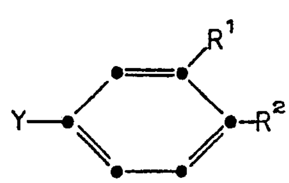

worin Y für $-NH_2$ oder -NCO steht, und $R^1$ und $R^2$ wie oben definiert sind, mit einer Sulfonylverbindung der Formel

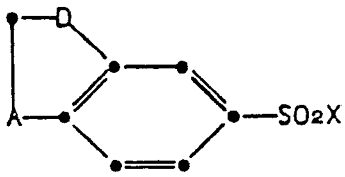

umgesetzt wird, worin X für -NCO, $-NH_2$ oder $-NH-COOR_3$ steht, wobei $R_3$ für $C_1$-$C_3$-Alkyl steht und A sowie D wie oben definiert sind, mit der Maßgabe, daß Y für $-NH_2$ steht, falls X -NCO oder $-NHCOOR_3$ ist, und daß Y für -NCO steht, falls X $-NH_2$ bedeutet.

11. Verwendung einer Verbindung der Formel (I)

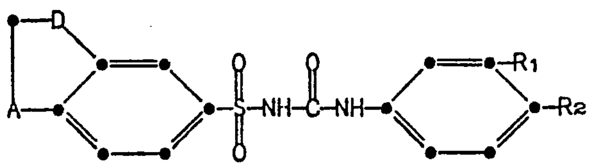

worin

A für -O-, $-NCH_3$, $-CH_2-$, $-CH_2CH_2-$ oder $-CH_2O-$ steht,
D für $-CH_2-$ oder -O- steht,
$R_1$ Wasserstoff oder Halogen ist, und
$R_2$ Halogen oder Trifluormethyl bedeutet,
zur Herstellung eines antitumoralen Arzneimittels.

**Patentansprüche für den Vertragsstaat:** AT

1. Verfahren zur Herstellung einer Verbindung der Formel (I)

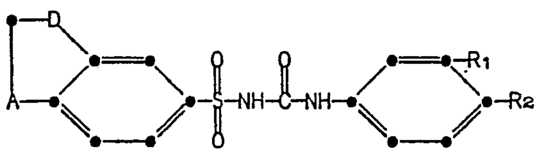
(I)

worin

A für -O-, $-NCH_3$, $-CH_2-$, $-CH_2CH_2-$ oder $-CH_2O-$ steht,
D für $-CH_2-$ oder -O- steht,
$R_1$ Wasserstoff oder Halogen ist, und
$R_2$ Halogen oder Trifluormethyl bedeutet,
mit der Maßgabe, daß $R_2$ nicht Chlor bedeutet, falls A Sauerstoff ist, D für $-CH_2-$ steht und $R_1$ Wasserstoff darstellt, dadurch gekennzeichnet, daß eine Verbindung der Formel

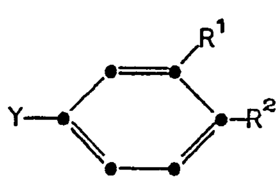

worin Y für $-NH_2$ oder -NCO steht, und $R^1$ und $R^2$ wie oben definiert sind, mit einer Sulfonylverbindung der Formel

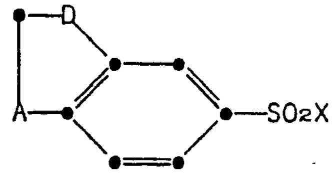

umgesetzt wird, worin X für -NCO, $-NH_2$ oder $-NH-COOR_3$ steht, wobei $R_3$ für $C_1$-$C_3$-Alkyl steht und A sowie D wie oben definiert sind, mit der Maßgabe, daß Y für $-NH_2$ steht, falls X -NCO oder $-NHCOOR_3$ ist, und daß Y für -NCO steht, falls X $-NH_2$ bedeutet.

2. Verfahren zur Herstellung einer Verbindung der Formel (I) gemäß Anspruch 1, nämlich der Formel

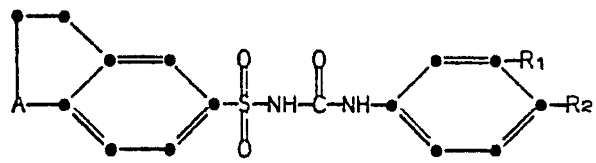

worin
A für $-CH_2-$ oder $-CH_2CH_2-$ steht,
$R_1$ Wasserstoff oder Halogen ist, und
$R_2$ Halogen oder Trifluoromethyl bedeutet.

3. Verfahren zur Herstellung einer Verbindung nach Anspruch 1 oder 2, dadurch gekennzeichnet, daß $R_1$ Wasserstoff ist.

4. Verfahren zur Herstellung einer Verbindung nach Anspruch 3, dadurch gekennzeichnet, daß $R_2$ Halogen ist.

5. Verfahren zur Herstellung einer Verbindung nach Anspruch 4, dadurch gekennzeichnet, daß $R_2$ Chlor ist.

6. Verfahren nach Anspruch 1, dadurch gekennzeichnet, daß hiernach folgende Verbindungen hergestellt werden:
N-([(4-Chlorophenyl)amino]carbonyl)-2,3-dihydro-1H-inden-5-sulfonamid,
N-([(4-Chlorphenyl)amino]carbonyl-5,6,7,8-tetrahydro-2-naphthalinsulfonamid oder
N-([(4-Chlorphenyl)amino]carbonyl)-1,3,-benzodioxol-5-sulfonamid.

**Revendications pour les Etas contractants:**
BE, CH, DE, FR, GB, IT, LI, LU, NL, SE

1. Composé de formule (I):

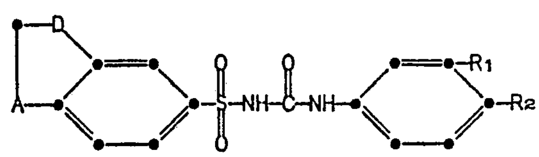
(I)

dans laquelle

A représente -O-, $-NCH_3$, $-CH_2-$, $-CH_2CH_2-$, ou $-CH_2O-$;

D représente $-CH_2-$ ou -O-;

$R_1$ représente un atome d'hydrogène ou un atome d'halogène, et

$R_2$ représente un atome d'halogène ou un groupe trifluorométhyle;

avec cette réserve que, lorsque A représente un atome d'oxygène, D représente $-CH_2-$ et $R_1$ représente un atome d'hydrogène, $R_2$ ne représente pas un atome de chlore.

2. Composé de formule (I), selon la revendication 1, ce composé étant

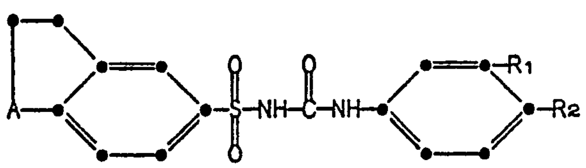

dans lequel

A représente $-CH_2-$ ou $-CH_2CH_2-$,

$R_1$ représente un atome d'hydrogène ou un atome d'halogène, et

$R_2$ représente un atome d'halogène ou un groupe trifluorométhyle.

3. Composé selon la revendication 1 ou 2, dans lequel $R_1$ représente un atome d'hydrogène.

4. Composé selon la revendication 3, dans lequel $R_2$ représente un atome d'halogène.

5. Composé selon la revendication 4, dans lequel $R_2$ représente un atome de chlore.

6. N-([(4-chlorophényl)amino]carbonyl)-2,3-dihydro-1H-indène-5-sulfonamide,

N-([(4-chlorophényl)amino]carbonyl-5,6,7,8-tétrahydro-2-naphtalènesulfonamide, ou

N-([(4-chlorophényl)-amino]carbonyl)-1,3-benzodioxole-5-sulfonamide.

7. Composé de formule (I), telle que définie dans la revendication 1, utilisé comme agent thérapeutique.

8. Composé de formule (I), telle que définie dans la revendication 1, utilisé comme agent antinéoplastique.

9. Formulation pharmaceutique comprenant, comme ingrédient actif, un composé de formule (I), telle que définie dans la revendication 1, en association avec un support, un diluant ou un excipient pharmaceutiquement acceptable, pour cette formulation.

10. Procédé de préparation d'un composé de formule (I), telle que définie dans la revendication 1, ce procédé consistant à faire réagir, avec un composé de formule:

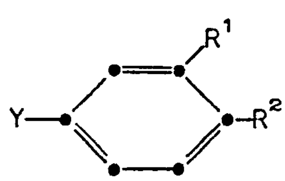

dans laquelle Y représente $-NH_2$ ou -NCO, tandis que $R_1$ et $R_2$ ont les significations définies ci-dessus, un composé sulfonyle de formule:

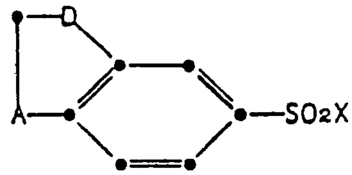

dans laquelle X représente -NCO, $-NH_2$ ou $-NH-COOR_3$, où $R_3$ représente un groupe alkyle en $C_1$-$C_3$, tandis que A et D ont les significations définies ci-dessus, avec cette réserve que, si X représente -NCO ou $-NHCOOR_3$, alors Y représente $-NH_2$, tandis que si X représente $-NH_2$, alors Y représente -NCO.

11. Utilisation d'un composé de formule (I)

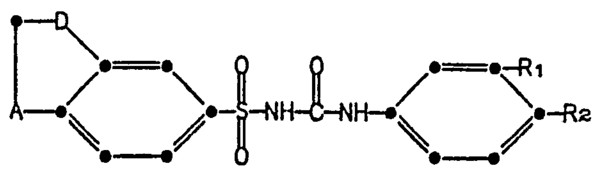

dans laquelle

A représente -O-, $-NCH_3$, $-CH_2-$, $-CH_2CH_2-$, ou $-CH_2O-$;

D représente $-CH_2-$ ou -O-;

$R_1$ représente un atome d'hydrogène ou un atome d'halogène, tandis que $R_2$ représente un atome halogène ou un groupe trifluorométhyle, pour la préparation d'un médicament antitumoral.

**Revendications pour l'Etat contractant:** AT

1. Procédé de préparation d'un composé de formule (I):

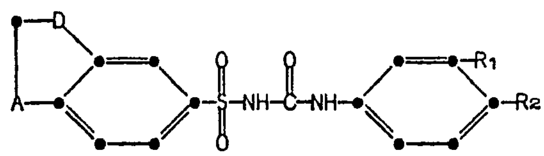 (I)

dans laquelle

A représente -O-, $-NCH_3$, $-CH_2-$, $-CH_2CH_2-$, ou $-CH_2O-$;

D représente $-CH_2-$ ou -O-;

$R_1$ représente un atome hydrogène ou un atome d'halogène, et

$R_2$ représente un atome d'halogène ou un groupe trifluorométhyle,

avec cette réserve que, lorsque A représente un atome d'oxygène, D représente $-CH_2-$ et $R_1$ représente un atome d'hydrogène, $R_2$ ne représente pas un atome de chlore, ce procédé consistant à faire réagir, avec un composé de formule:

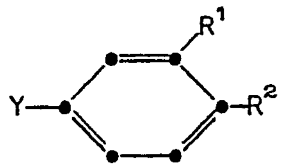

dans laquelle Y représente $-NH_2$ ou -NCO, tandis que $R_1$ et $R_2$ ont les significations définies ci-dessus, un composé sulfonyle de formule:

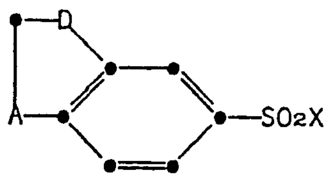

dans laquelle X représente -NCO, $-NH_2$ ou $-NH-COOR_3$, où $R_3$ représente un groupe alkyle en $C_1$-$C_3$, tandis que A et D ont les significations définies ci-dessus, avec cette réserve que, si X représente -NCO ou $-NHCOOR_3$, alors Y représente $-NH_2$, tandis que si X représente $-NH_2$, alors Y représente -NCO.

2. Procédé de préparation d'un composé de formule (I), selon la revendication 1, ce composé étant

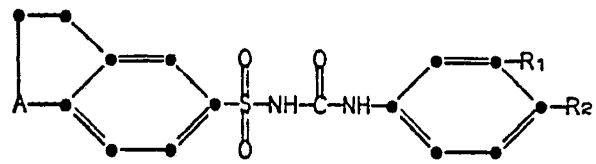

dans lequel

A représente $-CH_2-$ ou-$CH_2CH_2-$,

$R_1$ représente un atome d'hydrogène ou un atome d'halogène, et

$R_2$ représente un atome d'halogène ou un groupe trifluorométhyle.

3. Procédé de préparation d'un composé selon la revendication 1 ou 2, dans lequel $R_1$ représente un atome d'hydrogène.

4. Procédé de préparation d'un composé selon la revendication 3, dans lequel $R_2$ représente un atome d'halogène.

5. Procédé de préparation d'un composé selon la revendication 4, dans lequel $R_2$ représente un atome de chlore.

6. Procédé selon la revendication 1, ce procédé consistant à préparer le N-([(4-chlorophényl)amino]-carbonyl)-2,3-dihydro-1H-indène-5-sulfonamide,

le N-([(4-chlorophényl)amino]carbonyl-5,6,7,8--tétrahydro-2-naphtalènesulfonamide, ou

le N-([(4-chlorophényl)-amino]carbonyl)-1,3-benzodioxole-5-sulfonamide.